# FASCICULE DE BREVET EUROPEEN

(11) **EP 2 367 581 B1**
(45) Date de publication et mention de la délivrance du brevet: **05.09.2012**
(21) Numéro de dépôt: 09801476.4
(22) Date de dépôt: 02.12.2009
(51) Int. Cl.: A61M 5/20, A61D 7/00, F04B 13/00

(54) **POMPE VOLUMETRIQUE REGLABLE**
VERSTELLBARE DRUCKPUMPE
ADJUSTABLE POSITIVE-DISPLACEMENT PUMP

(30) Priorité: 19.12.2008 FR 0807153
(43) Date de publication de la demande: 28.09.2011
(73) Titulaire: Genia, 44680 Saint Hilaire De Chaleons (FR)
(72) Inventeur: COMMINGES, Bernard, F-44200 Nantes (FR)
(74) Mandataire: Godineau, Valérie
(86) Numéro de dépôt international: PCT/FR2009/052375
(87) Numéro de publication internationale: WO 2010/076447

(56) Documents cités:
- AU-A1- 2003 236 471
- US-A- 3 517 668
- US-A- 4 564 360
- US-A- 4 643 059
- US-A1- 2004 200 861

## Description

La présente invention concerne un perfectionnement aux pompes volumétriques réglables du type pistolet.

Elle concerne plus particulièrement un perfectionnement aux pompes du type pistolet comprenant une crosse de pistolet formant poignée de saisie du pistolet et un canon de pistolet comportant au moins un réservoir de pompe fermé par un fond muni d'un orifice de vidange du réservoir, un piston monté coulissant axialement à l'intérieur du réservoir et des moyens de réglage de la course du piston en fonction de la quantité de liquide à injecter, ces moyens de réglage comprenant une pièce rotative munie d'une pluralité de butées décalées angulairement autour de l'axe de rotation de ladite pièce et positionnables sélectivement, par rotation de ladite pièce, en regard d'une saillie radiale externe équipant ledit piston, chaque butée formant, à l'état positionné en regard de ladite saillie de piston, lors d'un déplacement axial du piston en direction du fond du réservoir, une butée d'arrêt du piston par contact d'appui de la saillie du piston contre ladite butée d'arrêt.

Un tel exemple de pompe est décrit dans le brevet AU-2003 236471. Dans ce brevet antérieur, la pièce rotative porte-butées est disposée à une extrémité du canon et est montée en rotation autour d'un axe parallèle à l'axe longitudinal du piston. Cette disposition implique que la pièce rotative est au contact immédiat de la poignée du pistolet de sorte qu'une rotation intempestive de la pièce porte-butées par frottement de la main de l'opérateur contre ladite pièce est possible. Il en résulte une possibilité de dérèglement du dosage dont l'opérateur ne s'aperçoit pas toujours. Le deuxième inconvénient de cette disposition est que, comme l'échelle de dosage est répartie sur tout le pourtour de la pièce rotative porte-butées et n'est pas entièrement visible d'un seul coup d'oeil, l'opérateur doit regarder les flancs du pistolet avant de procéder à la rotation de la pièce porte-butées pour un changement de dose. Ce qui entraîne une perte de temps.

D'autres exemples de pompe sont décrits notamment à travers les brevets US 2004/200861, US-4.564.360 et US-3.517.668.

Un but de la présente invention est donc de proposer une pompe volumétrique réglable du type pistolet dont la conception permet d'une part, de supprimer le risque d'injection de dose erronée résultant d'un déréglage intempestif du pistolet, d'autre part, de réduire le temps nécessaire à la sélection de la dose à injecter.

A cet effet, l'invention a pour objet une pompe volumétrique réglable, du type pistolet comprenant une crosse de pistolet formant poignée de saisie du pistolet et un canon de pistolet comportant au moins un réservoir de pompe fermé par un fond muni d'un orifice de vidange du réservoir, un piston monté coulissant axialement à l'intérieur du réservoir et des moyens de réglage de la course du piston en fonction de la quantité de liquide à injecter, ces moyens de réglage comprenant une pièce rotative munie d'une pluralité de butées décalées angulairement autour de l'axe de rotation de ladite pièce et positionnables sélectivement, par rotation de ladite pièce, en regard d'une saillie radiale externe équipant ledit piston, chaque butée formant, à l'état positionné en regard de ladite saillie de piston, lors d'un déplacement axial du piston en direction du fond du réservoir, une butée d'arrêt du piston par contact d'appui de la saillie du piston contre ladite butée d'arrêt, caractérisée en ce que la pièce porte-butées est disposée sur le canon du pistolet en une zone dite du dessus du canon opposée à la zone dite du dessous du canon solidaire de la crosse et est montée à rotation autour d'un axe orthogonal, de préférence perpendiculaire, à l'axe longitudinal du piston, la succession de butées d'arrêt disposées côte à côte de ladite pièce formant une surface d'allure spiralée s'enroulant autour de l'axe de rotation de ladite pièce.

Grâce à la disposition de la pièce porte-butées sur le dessus du canon, c'est-à-dire en un emplacement éloigné de la poignée de saisie du pistolet, le risque d'un déréglage de la dose sélectionnée par rotation intempestive de la pièce porte-butées à l'aide de la main tenant le pistolet est réduit. En outre, ce positionnement permet, en un seul coup d'oeil, de visualiser l'ensemble des doses et donc le sens de rotation à utiliser pour passer le plus rapidement possible d'une dose à une autre.

L'invention sera bien comprise à la lecture de la description suivante d'exemples de réalisation, en référence aux dessins annexés dans lesquels :

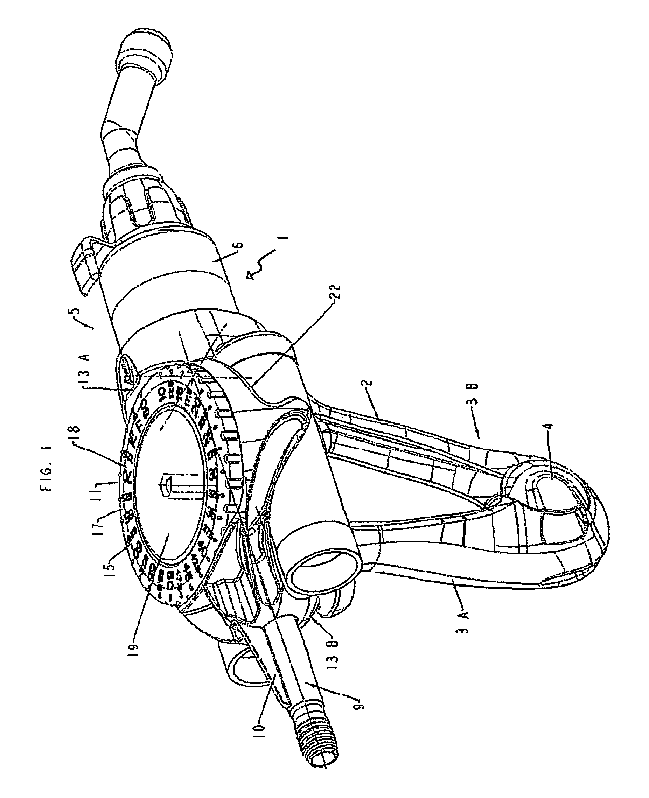

la figure 1 représente une vue en perspective en élévation d'un pistolet conforme à l'invention ;

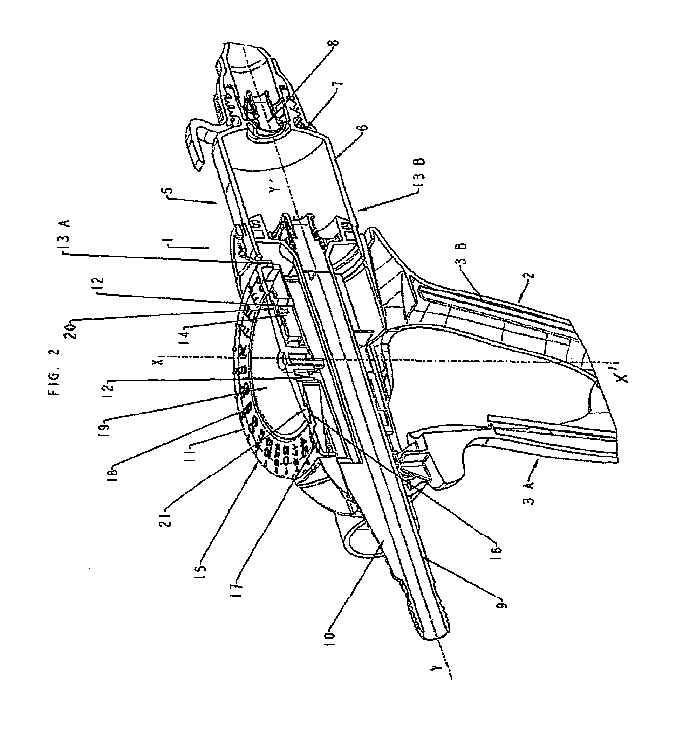

la figure 2 représente une vue en coupe longitudinale du pistolet de la figure 1 ;
les figures 3A et 3B représentent chacune une vue en perspective de la pièce porte-butées, l'une, correspondant à la figure 3A, prise côté repères de dosage de ladite pièce, l'autre, correspondant à la figure 3B, prise côté butées de ladite pièce.

Comme mentionné ci-dessus, le pistolet d'injection, objet de l'invention, comprend une crosse 2 de pistolet formant poignée de saisie du pistolet et un canon 5 de pistolet. Ce pistolet d'injection, encore appelé pistolet drogueur, est plus particulièrement destiné à l'injection de liquides aux animaux. Le canon 5 de pistolet comporte au moins un réservoir 6 de pompe de forme générale cylindrique. Ce réservoir est fermé par un fond 7 formant l'une des faces d'extrémité du corps cylindrique de réservoir. Ce fond 7 est muni d'un orifice 8 de vidange ou d'évacuation du fluide contenu dans le réservoir 6. Ici, l'orifice 8 de vidange est prolongé par une canule venant se raccorder par emboîtement audit orifice 8. Le canon comporte encore un piston 9 monté coulissant axialement à l'intérieur du réservoir 6. Ce piston 9 est introduit dans le réservoir par la face du réservoir opposée à celle fermée par le fond 7 de réservoir. Le fluide à injecter est donc contenu entre la tête de piston et le fond 7 muni de l'orifice 8 de vidange du réservoir 6. L'alimentation en fluide du réservoir s'opère de préférence par un alésage central du piston réalisé sous forme d'un corps creux. L'extrémité de piston opposée à la tête est donc à cet effet raccordable à un réservoir de liquide à injecter. A cet effet, ladite extrémité est filetée. Le réservoir 6 est quant à lui de préférence réalisé en plusieurs parties pour permettre un démontage d'une partie du réservoir et le remplacement de ce dernier. La partie non démontable du réservoir forme le support de la partie amovible dudit réservoir.

Dans les exemples représentés, le piston 9, monté coulissant axialement à l'intérieur du réservoir 6, est équipé de moyens d'actionnement formés par une poignée à deux branches 3A, 3B reliées l'une à l'autre par un axe 4 de pivotement. Ces branches 3A, 3B sont montées l'une, représentée en 3A aux figures, couplée au piston 9, tandis que l'autre, représentée en 3B aux figures, est formée par la crosse 2 du pistolet et solidaire du canon du pistolet. Ces branches 3A, 3B, qui forment les ailes d'un oiseau, sont rappelées dans une position dite de recul maximal du piston correspondant à un déplacement du piston en direction de l'extérieur du réservoir par des moyens de rappel appropriés non représentés. Le canon 5 de pistolet comporte encore des moyens de réglage de la course du piston 9 en fonction de la quantité de liquide à injecter. Ces moyens de réglage comprennent une pièce 11 rotative munie d'une pluralité de butées 12 décalées angulairement autour de l'axe XX' de rotation de ladite pièce 11 et positionnables sélectivement, par rotation de ladite pièce 11, en regard d'une saillie 10 radiale externe équipant ledit piston 9. Chaque butée 12 forme, à l'état positionné en regard de ladite saillie 10 de piston 9, lors d'un déplacement axial du piston 9 en direction du fond 7 du réservoir 6, une butée d'arrêt du piston 9 par contact d'appui de la saillie 10 du piston contre ladite butée d'arrêt. Lesdites butées d'arrêt sont donc destinées à limiter la course de déplacement axial du piston 9 en direction de l'intérieur du réservoir encore appelé avance du piston. Chaque butée correspond à une course de piston distincte d'une butée à une autre et donc à une dose d'injection.

De manière caractéristique à l'invention, la pièce 11 porte-butées est disposée sur le canon 5 du pistolet en une zone 13A dite du dessus du canon opposée à la zone 13B dite du dessous du canon solidaire de la crosse 2 et est montée à rotation autour d'un axe XX' orthogonal, de préférence perpendiculaire à l'axe YY' longitudinal du piston 9. La succession de butées 12 disposées côte à côte de ladite pièce forme une surface 14 d'allure spiralée s'enroulant autour de l'axe XX' de rotation de ladite pièce. Par surface d'allure spiralée, on entend dans ce qui précède, comme dans ce qui suit, que les butées sont montées, d'une butée à une autre, à écartement croissant de l'axe de rotation de la pièce porte-butées depuis une extrémité en direction de l'autre extrémité de l'enroulement formé.

La surface d'allure spiralée formée peut être continue lorsque les butées 12 sont disposées côte à côte de manière jointive ou discontinue lorsque les butées 12 sont disposées côte à côte mais écartées l'une de l'autre.

Dans les exemples représentés, la surface 14 d'allure spiralée délimitant une succession de butées de la pièce 11 porte-butées est une surface 14 étagée en escalier se développant d'une extrémité à l'autre de l'enroulement formé par ladite surface 14 d'allure spiralée, chaque marche de l'escalier étant apte à former une butée 12 d'arrêt du piston 9.

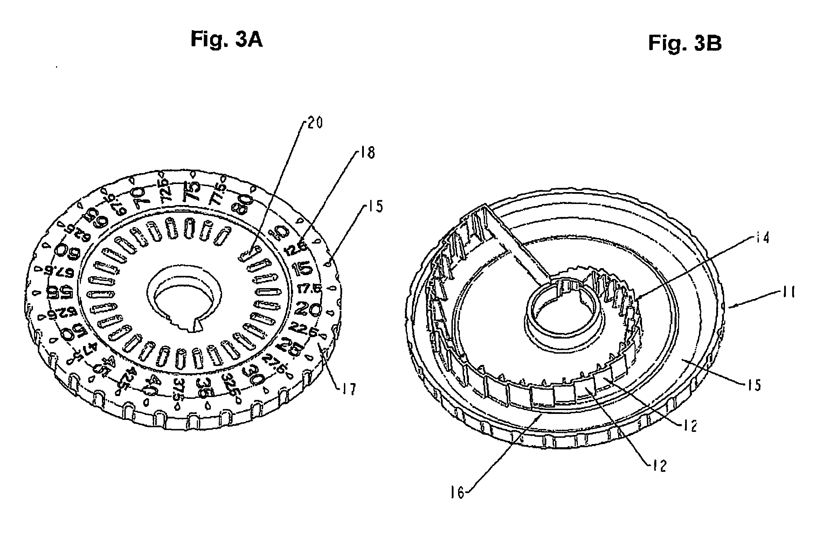

La pièce 11 porte-butées peut affecter un grand nombre de formes. De préférence, et conformément aux figures 3A et 3B, la pièce 11 porte-butées formant une partie du dessus du canon de pistolet comprend une platine 15 rotative portant, côté face 16 d'appui de la platine 15 sur le reste du canon, la surface 14 d'allure spiralée ménageant la succession de butées d'arrêt et, sur sa face 17 opposée, visible par l'opérateur en position d'injection, des repères 18 correspondant chacun à une dose d'injection 15.

Les repères 18 peuvent être constitués par des graduations ou tout autre marquage. La platine 15 est circulaire et évidée centralement pour le passage d'un axe de rotation de la pièce 11 porte-butées, les repères 18 étant disposés chacun sur un rayon de ladite platine 15. Ainsi, le passage d'une dose à une autre s'effectue par simple rotation de la pièce 11 porte-butées par la main de l'opérateur ne tenant pas la poignée de saisie. Par ailleurs, la main tenant la poignée de saisie est suffisamment éloignée de cette pièce 11 porte-butées pour empêcher tout déplacement intempestif de la pièce 11 porte-butées par ladite main. Cet entraînement en rotation de la pièce 11 porte-butées peut s'opérer en maintenant le pistolet en position d'injection. Dans l'exemple représenté, la platine est disposée dans un plan sensiblement orthogonal à l'axe longitudinal de la crosse 2 et orthogonalement, de préférence perpendiculairement, à l'axe longitudinal du piston. La surface 14 d'allure spiralée est formée par l'une des faces d'une paroi d'allure spiralée portée par ladite platine, cette paroi s'étendant sensiblement orthogonalement au plan de ladite platine 15. C'est ce mode de réalisation qui est représenté aux figures. Dans les figures, la platine est en outre pourvue d'un rebord périphérique externe venant se loger dans un évidement circulaire de forme complémentaire ménagé sur le dessus du canon. Ce rebord est de hauteur inférieure à la hauteur de la paroi d'allure spiralée pour permettre la venue en appui contre la face active de ladite paroi, c'est-à-dire la face opposée à celle tournée vers l'axe de rotation, de la saillie 10 du piston 9. Cette saillie 10 du piston 9 est ici réalisée sous forme d'une nervure du piston. L'axe de rotation de la pièce 11 porte-butées est porté par un couvercle 19 venant à recouvrement partiel de ladite platine 15 à l'état solidarisé du couvercle au canon. Généralement, le couvercle est encliqueté, par son axe formant axe de rotation, au canon du pistolet. La platine se trouve ainsi interposée entre couvercle et dessus du canon. Le couvercle n'a pas été représenté aux figures 3A et 3B. Le couvercle permet donc de maintenir en position d'appui la platine sur le dessus du canon.

Dans les figures, la pièce 11 porte-butées est équipée d'une série de crans 20 d'immobilisation en rotation de ladite pièce 11, chaque cran 20 étant associé à une butée 12 et étant apte à passer d'un mode inactif à un mode actif, à l'état positionné de ladite butée 12 associée en regard de la saillie 10 radiale externe du piston 9. Lesdits crans 20 sont formés par une succession de logements ménagés dans ladite platine 15, lesdits logements coopérant tour à tour avec une languette 21 ou saillie stationnaire de la pompe. Les logements de forme oblongue sont tous disposés sur un cercle centré sur l'axe de rotation de la pièce 11. Ces logements peuvent être borgnes ou traversants. Lors de la rotation de la platine, les logements défilent devant la languette 21 stationnaire jusqu'à ce que le logement correspondant à la graduation sélectionnée se positionne en regard de la languette qui pénètre dans ledit logement. Lors de l'entraînement en rotation de la platine, la languette se déforme élastiquement pour ne pas nuire à la rotation de la platine et passe d'un logement à un autre.

Enfin, le canon 5 de réservoir comporte, au droit de la pièce 11 porte butées réalisée circulaire, en deux points diamétralement opposés de ladite pièce 11, un compartiment 22 de rangement réalisé dans l'épaisseur dudit canon. Chaque compartiment 22 peut servir de rangement d'un organe, tel qu'un crayon, de marquage des animaux.

Le fonctionnement de la pompe, telle que décrite ci-dessus, est analogue à l'état de la technique. On procède dans un premier temps au choix de la dose à injecter par rotation de la platine jusqu'à positionnement d'un repère de la platine en regard d'un repère de référence stationnaire disposé de préférence sur le dessus du canon au droit de la saillie 10 du piston 9. On actionne le piston par rapprochement desdites branches 3A, 3B de la poignée jusqu'à une position en butée de la saillie 10 du piston 9 contre une butée de la pièce porte-butée. Les branches de la poignée sont relâchées pour revenir en position écartée. La pompe est donc prête pour une nouvelle injection. Si une nouvelle dose doit être choisie, il suffit à l'opérateur, à l'aide de sa main libre, de venir tourner la platine jusqu'à sélection d'une nouvelle dose puis de procéder immédiatement à cette injection. L'opérateur peut donc, à l'aide de son autre main, maintenir le pistolet en position d'injection. Ainsi, le passage d'une dose à une autre peut s'opérer extrêmement rapidement. En outre, comme la platine est entièrement montée libre à rotation sur 360°, le passage de la dose la plus élevée à la dose la plus faible peut s'effectuer de manière quasi instantanée. Il n'est pas nécessaire de revenir à une position initiale. La platine est donc montée à rotation sans fin.

## Revendications

1. Pompe volumétrique réglable, du type pistolet (1) comprenant une crosse (2) de pistolet formant poignée de saisie du pistolet et un canon (5) de pistolet comportant au moins un réservoir (6) de pompe fermé par un fond (7) muni d'un orifice (8) de vidange du réservoir (6), un piston (9) monté coulissant axialement à l'intérieur du réservoir et des moyens de réglage de la course du piston (9) en fonction de la quantité de liquide à injecter, ces moyens de réglage comprenant une pièce (11) rotative munie d'une pluralité de butées (12) décalées angulairement autour de l'axe (XX') de rotation de ladite pièce (11) porte-butées et positionnables sélectivement, par rotation de ladite pièce (11) porte-butées, en regard d'une saillie (10) radiale externe équipant ledit piston (9), chaque butée (12) formant, à l'état positionné en regard de ladite saillie (10) de piston (9), lors d'un déplacement axial du piston (9) en direction du fond (7) du réservoir (6), une butée d'arrêt du piston (9) par contact d'appui de la saillie (10) du piston contre ladite butée d'arrêt,
**caractérisée en ce que** la pièce (11) porte-butées est disposée sur le canon (5) du pistolet en une zone (13A) dite du dessus du canon (5), opposée à la zone (13B) dite du dessous du canon (5) solidaire de la crosse (2), et est montée à rotation autour d'un axe (XX') orthogonal, de préférence perpendiculaire à l'axe (YY') longitudinal du piston (9), la succession de butées (12) disposées côte à côte de ladite pièce (11) porte-butées formant une surface (14) d'allure spiralée s'enroulant autour de l'axe (XX') de rotation de ladite pièce (11) porte-butées.

2. Pompe volumétrique réglable selon la revendication 1,
**caractérisée en ce que** la surface (14) d'allure spiralée délimitant une succession de butées (12) de la pièce (11) porte-butées est une surface (14) étagée en escalier se développant d'une extrémité à l'autre de l'enroulement formé par ladite surface (14) d'allure spiralée, chaque marche de l'escalier étant apte à former une butée (12) d'arrêt du piston (9).

3. Pompe volumétrique réglable selon l'une des revendications 1 et 2,
**caractérisée en ce que** la pièce (11) porte-butées formant une partie du dessus du canon (5) de pistolet comprend une platine (15) rotative portant, côté face (16) d'appui de la platine (15) sur le reste du canon, la surface (14) d'allure spiralée ménageant la succession de butées d'arrêt et, sur sa face (7) opposée, visible par l'opérateur en position d'injection, des repères (18) correspondant chacun à une dose d'injection (15).

4. Pompe volumétrique réglable selon la revendication 3,
**caractérisée en ce que** la platine (15) est disposée dans un plan sensiblement orthogonal à l'axe longitudinal de la crosse (2) et **en ce que** la surface (14) d'allure spiralée est formée par l'une des faces d'une paroi d'allure spiralée portée par ladite platine (15), cette paroi s'étendant sensiblement orthogonalement au plan de ladite platine (15).

5. Pompe volumétrique réglable selon l'une des revendications 3 et 4,
**caractérisée en ce que** la platine (15) est circulaire et évidée centralement pour le passage d'un axe de rotation de la pièce (11) porte-butées, les repères (18) étant disposés chacun sur un rayon de ladite platine (15).

6. Pompe volumétrique réglable selon la revendication 5,
**caractérisée en ce que** l'axe de rotation de la pièce (11) porte-butées est porté par un couvercle (19) venant à recouvrement partiel de ladite platine (15).

7. Pompe volumétrique réglable selon l'une des revendications 1 à 6,
**caractérisée en ce que** la pièce (11) porte-butées est équipée d'une série de crans (20) d'immobilisation en rotation ladite pièce (11) porte-butées, chaque cran (20) étant associé à une butée (12) et étant apte à passer d'un mode inactif à un mode actif, à l'état positionné de ladite butée (12) associée en regard de la saillie (10) radiale externe du piston (9).

8. Pompe volumétrique réglable selon la revendication 7,
**caractérisée en ce que** lesdits crans (20) sont formés par une succession de logements ménagés dans ladite platine (15), lesdits logements coopérant tour à tour avec une languette (21) ou saillie stationnaire de la pompe.

9. Pompe volumétrique réglable selon l'une des revendications 1 à 8,
**caractérisée en ce que** le canon (5) de réservoir comporte, au droit de la pièce (11) porte butées réalisée circulaire, en deux points diamétralement opposés de ladite pièce (11) porte-butées, un compartiment (22) de rangement réalisé dans l'épaisseur dudit canon.

10. Pompe volumétrique réglable selon l'une des revendications 1 à 9,
**caractérisée en ce que** le piston (9) monté coulissant axialement à l'intérieur du réservoir (6) est équipé de moyens d'actionnement formés par une poignée à deux branches (3A, 3B) reliées l'une à l'autre par un axe (4) de pivotement, ces branches (3A, 3B) étant montées l'une (3A) couplée au piston (9), l'autre (3B), formée par la crosse (2) du pistolet, solidaire du canon (5) du pistolet.

## Claims

1. An adjustable positive-displacement pump, of the gun (1) type comprising a gun grip (2) forming a gripping handle of the gun and a gun barrel (5) including at least one pump reservoir (6) closed by a bottom (7) provided with an opening (8) for emptying the reservoir (6), a piston (9) axially slidingly mounted inside the reservoir, and means for adjusting the travel of the piston (9) as a function of the quantity of liquid to be injected, said adjustment means comprising a rotary part (11) provided with a plurality of stops (12) angularly offset around the axis (XX') of rotation of said stop-bearing part (11) and which can be selectively positioned, by rotating said stop-bearing part (11), across from an outer radial protrusion (10) equipping said piston (9), each stop (12) forming, in its state positioned across from said piston (9) protrusion (10), during an axial movement of the piston (9) toward the bottom (7) of the reservoir (6), a stop stopping the piston (9) through bearing contact of the protrusion (10) of the piston against said stopping stop, **characterized in that** the stop-bearing part (11) is positioned on the barrel (5) of the gun at a so-called top area (13A) of the barrel (5), opposite the so-called bottom area (13B) of the barrel (5) integral with the grip (2), and is rotatably mounted around an axis (XX') that is orthogonal, preferably perpendicular to the longitudinal axis (YY') of the piston (9), the series of stops (12) positioned side by side with the stop-bearing portion (11) forming a spiral surface (14) winding around the axis (XX') of rotation of said stop-bearing part (11).

2. The adjustable positive-displacement pump according to claim 1,
**characterized in that** the spiral surface (14) defining a series of stops (12) of the stop-bearing part (11) is a stair-stepped surface (14) developing from one end to the other of the winding formed by said spiral surface (14), each step of the stairs being able to form a stop (12) for stopping the piston (9).

3. The adjustable positive-displacement pump according to one of claims 1 and 2,
**characterized in that** the stop-bearing part (11) forming a portion of the top of the gun barrel (5) comprises a rotary platen (15) bearing, on the side of the bearing surface (16) of the platen (15) on the rest of the barrel, the spiral surface (14) forming the series of stops and, on its opposite surface (7), visible by the operator in the injection position, references (18) each corresponding to an injection dose (15).

4. The adjustable positive-displacement pump according to claim 3,
**characterized in that** the platen (15) is arranged in a plane substantially orthogonal to the longitudinal axis of the grip (2) and **in that** the spiral surface (14) is formed by one of the surfaces of a spiral wall supported by said platen (15), said wall extending substantially orthogonally to the plane of said platen (15).

5. The adjustable positive-displacement pump according to one of claims 3 and 4,
**characterized in that** the platen (15) is circular and hollowed out in the middle for the passage of an axis of rotation of the stop-bearing part (11), the references (18) each being arranged on a radius of said platen (15).

6. The adjustable positive-displacement pump according to claim 5,
**characterized in that** the axis of rotation of the stop-bearing part (11) is supported by a lid (19) partially covering said platen (15).

7. The adjustable positive-displacement pump according to one of claims 1 to 6,
**characterized in that** the stop-bearing part (11) is equipped with a series of notches (20) for rotationally immobilizing said stop-bearing part (11), each notch (20) being associated with a stop (12) and being able to go from an inactive mode to an active mode, in the state of said associated stop (12) positioned across from the outer radial protrusion (10) of the piston (9).

8. The adjustable positive-displacement pump according to claim 7,
**characterized in that** said notches (20) are formed by a series of housings made in said platen (15), said housings cooperating in turn with a stationary tab (21) or protrusion of the pump.

9. The adjustable positive-displacement pump according to one of claims 1 to 8,
**characterized in that** the reservoir grip (5) has, at the circular stop-bearing part (11), at two diametrically opposite points of said stop-bearing part (11), a storage compartment (22) formed in the thickness of said grip.

10. The adjustable positive-displacement pump according to one of claims 1 to 9,
**characterized in that** the piston (9) axially slidingly mounted inside the reservoir (6) is equipped with actuating means formed by a handle with two branches (3A, 3B) connected to one another by a hinge pin, said branches (3A, 3B) being mounted one (3A) coupled to the piston (9), the other (3B), formed by the grip (2) of the gun, integral with the barrel (5) of the gun.

## Patentansprüche

1. Verstellbare Druckpumpe vom Typ Pistole (1), die einen Pistolenkolben (2), der einen Griff der Pistole bildet, und einen Pistolenlauf (5) umfasst, der mindestens einen Pumpen-Vorratsbehälter (6) aufweist, der von einem Boden (7) verschlossen wird, der mit einer Entleerungsöffnung (8) des Vorratsbehälters (6) ausgestattet ist, wobei ein Kolben (9) axial gleitend im Vorratsbehälter montiert ist, und Verstellmittel des Laufs des Kolbens (9) in Abhängigkeit von der einzuspritzenden Flüssigkeitsmenge, wobei diese Verstellmittel ein Drehteil (11) umfassen, das mit einer Vielzahl von Anschlägen (12) ausgestattet ist, die um die Drehachse (XX') des anschlagtragenden Teils (11) winklig versetzt und selektiv durch Drehen des anschlagtragenden Teils (11) gegenüber einem radialen externen Vorsprung (10), der den Kolben (9) ausstattet, positionierbar sind, wobei jeder Anschlag (12) im gegenüber dem Vorsprung (10) des Kolbens (9) positioniertem Zustand bei einer axialen Verschiebung des Kolbens (9) in Richtung des Bodens (7) des Vorratsbehälters (6) einen Stoppanschlag des Kolben (9) durch Abstützkontakt des Vorsprungs (10) des Kolbens auf dem Stoppanschlag bildet,
**dadurch gekennzeichnet, dass** das anschlagtragende Teil (11) auf dem Pistolenlauf (5) in einer oberen Zone (13A) des Laufs (5) angeordnet ist, die der unteren Zone (13B) des Laufs (5), die mit dem Kolben (2) verbunden ist, gegenüberliegt, und um eine orthogonale Achse (XX'), vorzugsweise im rechten Winkel zur Längsachse (YY') des Kolbens (9), rotierend montiert ist, wobei die Abfolge von Anschlägen (12), die nebeneinander auf dem anschlagtragenden Teil (11) angeordnet sind, eine spiralige Fläche (14) bilden, die sich um die Drehachse (XX') des anschlagtragenden Teils (11) wickelt.

2. Verstellbare Druckpumpe nach Anspruch 1,
**dadurch gekennzeichnet, dass** die spiralige Fläche (14), die eine Abfolge von Anschlägen (12) des anschlagtragenden Teils (11) begrenzt, eine treppenförmig etagige Fläche (14) ist, die sich von einem Ende zum anderen der von der spiraligen Fläche (14) gebildeten Rolle entwickelt, wobei jede Treppenstufe imstande ist, einen Stoppanschlag (12) des Kolbens (9) zu bilden.

3. Verstellbare Druckpumpe nach einem der Ansprüche 1 und 2,
**dadurch gekennzeichnet, dass** das anschlagtragende Teil (11), das einen oberen Abschnitt des Pistolenlaufs (5) bildet, eine drehende Platine (15) umfasst, die auf der Abstützseite (16) der Platine (15) auf dem restlichen Lauf die spiralige Fläche (14) trägt, die die Abfolge von Stoppanschlägen ausbildet und auf ihrer gegenüberliegenden Seite (7), die in Einspritzstellung vom Bediener sichtbar ist, Orientierungspunkte (18), die jeweils einer Einspritzdosis (15) entsprechen.

4. Verstellbare Druckpumpe nach Anspruch 3,
**dadurch gekennzeichnet, dass** die Platine (15) in einer zur Längsachse des Kolbens (2) etwa orthogonalen Ebene angeordnet ist und dass die spiralige Fläche (14) von einer der Seiten einer spiraligen Wand gebildet wird, die von der Platine (15) getragen wird, wobei sich diese Wand etwa orthogonal zur Ebene der Platine (15) erstreckt.

5. Verstellbare Druckpumpe nach einem der Ansprüche 3 und 4,
**dadurch gekennzeichnet, dass** die Platine (15) rund und in der Mitte für den Durchgang einer Drehachse des anschlagtragenden Teils (11) ausgehöhlt ist, wobei die Orientierungspunkte (18) jeweils auf einem Radius der Platine (15) angeordnet sind.

6. Verstellbare Druckpumpe nach Anspruch 5,
**dadurch gekennzeichnet, dass** die Drehachse des anschlagtragenden Teils (11) von einem Deckel (19) getragen wird, der die Platine (15) teilweise verdeckt.

7. Verstellbare Druckpumpe nach einem der Ansprüche 1 bis 6,
**dadurch gekennzeichnet, dass** das anschlagtragende Teil (11) mit einer Reihe von Kerben (20) zum Feststellen der Drehung des anschlagtragenden Teils (11) ausgestattet ist, wobei jede Kerbe (20) mit einem Anschlag (12) kombiniert ist und imstande, im positionierten Zustand des entsprechenden Anschlags (12) gegenüber dem radialen externen Vorsprung (10) des Kolbens (9) von einem inaktiven Modus in einen aktiven Modus zu wechseln.

8. Verstellbare Druckpumpe nach Anspruch 7,
**dadurch gekennzeichnet, dass** die Kerben (20) von einer Abfolge von Aufnahmen gebildet werden, die in die Platine (15) eingearbeitet sind, wobei die Aufnahmen jeweils mit einer Zunge (21) oder einem stationären Vorsprung der Pumpe zusammenarbeiten.

9. Verstellbare Druckpumpe nach einem der Ansprüche 1 bis 8,
**dadurch gekennzeichnet, dass** der Lauf (5) des Vorratsbehälters neben dem runden anschlagtragenden Teil (11) an zwei direkt gegenüberliegenden Punkten des anschlagtragenden Teils (11) ein Ablageabteil (22) aufweist, das in die Dicke des Laufs eingearbeitet ist.

10. Verstellbare Druckpumpe nach einem der Ansprüche 1 bis 9,
**dadurch gekennzeichnet, dass** der axial gleitend im Vorratsbehälter (6) montierte Kolben (9) mit Betätigungsmitteln ausgestattet ist, die von einem Griff mit zwei Armen (3A, 3B) gebildet werden, die miteinander durch eine Schwenkachse (4) verbunden sind, wobei einer (3A) dieser Arme (3A, 3B) am Kolben (9) befestigt ist und der andere (3B) vom Kolben (2) der Pistole gebildet wird, der mit dem Lauf (5) der Pistole verbunden ist.
